# EUROPEAN PATENT SPECIFICATION

(11) **EP 2 830 683 B1**
(45) Date of publication and mention of the grant of the patent: **29.08.2018**
(21) Application number: 13767759.7
(22) Date of filing: 29.03.2013
(51) Int. Cl.: A61M 5/31, A61B 10/00, A61B 10/02, A61M 5/178, A61M 5/165

(54) **NEEDLE FILTER APPARATUS**
NADELFILTERVORRICHTUNG
APPAREIL FILTRE D'AIGUILLE

(30) Priority: 29.03.2012 US 201261617077 P
(43) Date of publication of application: 04.02.2015
(73) Proprietor: Saint-Gobain Performance Plastics Corporation, Solon, OH 44139 (US)
(72) Inventor: LIN, Zhenwu, Pasadena, CA 91105 (US); ADRIAN, Kenneth, D., Manassas, VA 20111 (US); RENFREW, Kenneth, Huntersville, NC 28078 (US); ANDREWS, Jacob, Washington, DC 20011 (US)
(74) Representative: Zimmermann & Partner Patentanwälte mbB
(86) International application number: PCT/US2013/034653
(87) International publication number: WO 2013/149175

(56) References cited:
- EP-A1- 0 364 173
- EP-A1- 0 364 173
- WO-A1-99/22663
- WO-A1-99/22663
- CN-U- 201 987 980
- CN-Y- 200 951 242
- GB-A- 2 391 494
- US-A- 4 316 462
- US-A- 5 147 309
- US-A- 5 147 309
- US-A1- 2006 102 555
- US-A1- 2007 060 841
- US-A1- 2009 264 829

## Description

### FIELD OF THE DISCLOSURE

The disclosure relates generally to an accessory needle filter apparatus for filtering fluids and solutions aspirated into a syringe or like device. More particularly, the disclosure concerns disposable needle filter apparatus for attachment to syringes bearing needles prior to fluid/solution aspiration into the syringe.

### BACKGROUND OF THE DISCLOSURE

A common application of needle-bearing syringes is to draw medicament or drug-laden solutions into the syringes for administration into a human subject via subcutaneous, intra-muscular and/or intravenous injection. Hypodermic needles and the like are used to perform the tissue penetration and solution administration event. Often times, the solutions are prepared by dissolving a drug in pill, tablet or powder form into an aqueous solvent. The implements used to make the pharmaceutical preparations range from rather sophisticated lab apparatuses in aseptic conditions to more primitive arrangements in which a spoon or like device is used as a mixing vessel with or without the application of heat. In the latter setting, the possibility for solution contamination via environmental factors, e.g., sneezing, coughing, contaminating touching, is considerable.

Apart from the possibility of environmental contamination, the substances used to make the solutions may also pose contamination risks. Although pills and tablets and other powder-based medicaments and medications are designed for relatively safe administration: via the alimentary tract, infusion of the same pharmaceutical preparations via hypodermic needle and the like creates a different biological and physiological dynamic in which what is in one setting a safely ingested material becomes a potentially lethal contaminant.

As is well known in the art, drugs in one form or another, but particularly in pill form, are often prepared with waxes, chalky substances, binders, fillers and other additives to ease mechanical ingestion, or to control drug delivery release time, among other functions. Although innocuous substances in the alimentary tract, these same substances can easily be described as undesirable for hypodermic needle injection.

A yet further source of potential solution contamination is the use of needle-bearing syringes for recreational drug use. Apart from the various potential sources of contamination described herein, "needle sharing" adds a significant additional source of potentially lethal contamination. Although biological pathogens may be introduced at any stage of the solution preparation process, transmission of pathogens from one person to another is greatly increased with the use of the same solution with previously used needles.

An even more insidious source of potential contamination can occur in controlled environments, e.g., hospital and clinics, maintained to minimize the presence of pathogens in bacterial and/or viral form. As is commonly known, medications in fluid form including vaccines, often are stored in airtight containers or vials having caps with rubber membranes secured thereto. To obtain a dose of the enclosed medication, a syringe/needle assembly is used to extract the medication. The tip of the needle is pressed against the: membrane to pierce it so as to allow entry of the needle. As the needle is advanced, the resilient membrane seals around the needle to prevent air infiltration in one direction and fluid escape in the other.

Although the interior of the container and the medication itself may be sterile, the outside of the container may have surface contamination. For example, the container top may be contaminated by contact transmission of pathogens or via aerial assault, e.g., a health care professional sneezing in close proximity to the container. Microscopic pathogens may be deposited onto the container including the membrane without any visible sign of the contamination. Once the syringe needle is pressed against the membrane for insertion, any contamination on the membrane can be transferred to the outside of the needle and spread along the needle as the needle is advanced through, and retracted from the membrane. Although the contents drawn into the needle and syringe may be sterile and contaminant fee, the outside surface of the needle may harbor contamination obtained from the fluid extraction process.

Unknown to the healthcare professional handling the syringe, use of the same needle to penetrate the skin and tissue of a patient to administer the medication results in exposure of the patient's internal tissues to the contaminant. What is needed is a means to ensure solutions aspired into syringes for subsequent administration are effectively free of environmental and solution-borne contaminants. What is also needed is a means to ensure a needle used for insertion into human tissue to administer medications is free of pathogenic contamination both inside and outside the needle.

Many devices and methods have been developed to address these problems, however, the shortcomings and inefficiencies of these prior attempts are considerable. For example, U.S. Pat. No. 5, 125,415 discloses a syringe tip cap. The cap includes an enclosed filter for filtering air from a loaded syringe prior to administration. To use the syringe cap, a desired solution or fluid is drawn into the syringe via an attached hypodermic needle or the like. As is often the case, whatever is drawn into the syringe will include any contaminants in the solution including the aspiration of unwanted air, which, in and of itself, can be considered a contaminant, particularly if the drawn fluid is destined for later intravenous administration, or for lab testing when, for example, blood gas levels are measured if the fluid is drawn blood.

Once the syringe is loaded, the needle is removed and the cap is secured to the syringe. The plunger is then advanced down the syringe barrel while the syringe is held in a substantially vertical orientation with the connection end at the top. This allows lower density gases to migrate to the now top of the syringe where it enters the cap and passes through the enclosed filter. Once the air has been purged, the fluid contacts the filter and causes it to swell so as to seal the cap. This ensures the fluid is maintained in the syringe. The cap is then removed and the fluid is ready for expulsion from the syringe,

The drawbacks of this approach are significant and considerable. First, the cap, by design, does not allow for the flow of fluid through: the filter. This prevents the filter from being used to filter any non-gaseous contaminants out of the contained fluid. Second, the cap requires the needle to be removed to perform the filtration step and later re-attached, or replaced with a new needle for further use. This adds considerably to the possibility of a needle stick event due to the need to handle the needle portion multiple times for one use.

Another approach taken is disclosed in U.S. Pat No. 3,859,999. In the '999 patent, a single filament is rolled and intertwined into a wad. The wad is placed at the bottom of the syringe barrel or in a bore formed in a needle holder so as to place the wad between the connected fluid channels of the needle holder and the connection end of the syringe. This approach has a similar deficiency to that of the '415 patent in that the needle remains exposed throughout the series of steps taken to load the filter and operate the syringe. The chances for a possible needle stick event are numerous.

U.S. Pat. Appl. Publ. No. US 2008/0097353 discloses a filter secured in a distal end of a syringe needle having frangible sections. Fluid is drawn into the needle and attached syringe through the needle tip. The filter disposed behind the needle tip filters the incoming fluid. Once the fluid has been aspirated to the desired amount, the needle tip including the fitter is detached by snapping the needle at the frangible section located behind the filter. The needle tip and filter are thereafter discarded. The section of the needle remaining after the separation has a property shaped new tip for tissue penetration. Although this approach solves the problem of filtering fluids before entry into the syringe, the constantly exposed needle combined with the manual method used to remove the needle tip presents a constant danger of a needle stick event.

A yet further approach to solve contamination problems with aspirated fluids is shown in U.S. Pat. No. 4,066,079. The '079 patent discloses a filter apparatus that attaches to a syringe at one end, and to needle at the opposing end. The filter apparatus defines a pair of chambers, each having a one-way check valve. One valve is dedicated to permit fluid to flow into the syringe, while the other is dedicated to allow fluid to flow out of the syringe. A filter is lodged behind the outward bound check valve to filter the contained fluid as it flows out of the syringe. Although this system requires less manipulation of the needle, it still presents the problem of an exposed needle with the possibility of a needle prick event. Moreover, part of the chamber through which the fluid is drawn into the filter apparatus contacts the fluid after filtration, but before entry into the hilt of the needle. Any contaminants deposited in the shared chamber can be reintroduced into the fluid as it exits the apparatus into the needle. This counteracts and defeats the purpose of the filter secured in the apparatus to eliminate contaminants before injection of the intended fluid.

A still further approach is disclosed in U.S. Pat. Appl. Publ. No. 2009/0264829 in which a needle sheath with a filter plug secured to a distal end is disclosed. In this apparatus, the sheath encloses the needle and allows for the aspiration of fluid through the filter and into the needle. In one embodiment, the needle tip enters the filter and receives fluid directly through the filter. The tight-fitting sheath allows for the creation of a vacuum in the sheath to promote fluid flow into the needle and syringe. Once the syringe is loaded, the sheath is removed and the syringe is ready for use. Although this approach solves the problem of an exposed needle, it requires the sheath to be fitted to the specific syringe and needle, which can differ considerably with respect to syringe barrel width as well as needle length. Moreover, the design allows for the needle to pierce the filter and has no means to prevent the extent to which the needle pierces the filter U.S. Pat. Appl. Publ. No. 5147309 A teaches an apparatus for retaining hazardous fluid ejected during priming a needle by purging air from a syringe. The apparatus comprises a gas permeable hydrophobic filter disposed in the containment portion containing the needle during priming.

What is needed and what is provided herein is a universal needle filter assembly that effectively eliminates fluid contaminants from being drawn into a syringe and protects against needle stick events regardless the size and structure of the syringe and/or needle. What is further needed is a disposable needle filter assembly that includes features to completely enclose a syringe needle and a means to create a substantially airtight chamber to allow for the effective aspiration of fluid through the filter and into the syringe in an aseptic, substantially pathogen-free and contaminant-free manner. What is also needed is a needle filter assembly that incorporates a needle stop surface to prevent a syringe needle from penetrating the needle filter when engaged with the needle filter assembly. These and other objects of the disclosure will become apparent from a reading of the following summary and detailed description of the disclosure as well as a review of the appended drawings.

### SUMMARY OF THE DISCLOSURE

In one aspect of the disclosure, a needle filter apparatus for filtering fluids and solutions aspirated into a syringe comprises a filter housing having a first end and a second end and having portions defining a filter chamber; a filter media secured in the filter chamber; an inlet port extending from the filter housing wherein the inlet port has portions defining a channel in fluid communication with the filter chamber; a syringe-receiving neck extending from the second end of the filter housing, wherein the neck has portions defining a neck chamber in fluid communication with the filter chamber; a needle stop shoulder secured to, or formed in, the neck chamber, wherein the shoulder extends radially inwardly from the neck inner wall so as to intersect a centerline of the neck chamber and to provide a mechanical barrier to needle travel beyond the shoulder, and wherein the shoulder has a first end that extends downwardly toward, and in proximity to, the filter housing so as to create a gap between the shoulder and the filter housing, and a second end that extends upwardly along the neck inner wall to form a stopper registration surface; and, a needle-sealing stopper secured in the receiving neck, wherein the stopper is configured to register against the stopper registration surface.

In another aspect of the disclosure, a needle filter apparatus for filtering fluids and solutions aspirated into a syringe comprises: a filter housing having a first end and a second end and having portions defining a filter chamber; a filter media secured in the filter chamber; a syringe-receiving neck extending from the second end of the filter housing, wherein the neck has an inner wall defining a neck chamber in fluid communication with the filter chamber; a needle stop shoulder secured to, or formed in, the neck chamber, wherein the needle stop comprises a needle strike plate suspended substantially centrally within the neck chamber between a plurality of spacer columns and functioning as a needle stop for a syringe needle of the syringe, wherein the spacer columns are secured to, or integral with, the neck inner wall, wherein each of the plurality of spacer columns comprise a bottom end extending downwardly from the strike plate that collectively form a registration surface for registration against portions of the filter housing, and a top end extending upwardly from the strike plate that collectively form a registration surface for a needle-sealing stopper, wherein the bottom ends collectively create a gap between the filter housing and a bottom of the strike plate, and the top ends collectively create a gap between the needle-sealing stopper and a top surface of the strike plate, and wherein the substantially central location of the strike plate prevents a needle from passing beyond the needle stop regardless of the angle or orientation of needle insertion into the apparatus; and the needle-sealing stopper secured in the syringe-receiving neck.

The needle filter apparatus may include a modular syringe receiving neck. The receiving neck may be configured to accommodate different sized syringe bodies. An elongate neck may be provided and configured with stepped tapered segments of serially larger cross-sectional diameters to accommodate differently sized syringes. These and other aspects of the disclosure will become apparent from a review of the appended drawings and a reading of the following detailed description of the disclosure.

### BRIEF DESCRIPTION OF THE DRAWINGS

FIG. 1 is a side partial sectional view in partial phantom of a truncated needle filter apparatus according to one embodiment of the disclosure.
FIG. 2 is a side partial sectional view in partial phantom of the truncated needle filter apparatus shown in FIG. 1 with a needle inserted into the apparatus.
FIG. 3 is a top sectional view of a needle filter syringe receiving neck with a needle stop shoulder according to multiple embodiments of the disclosure.
FIG. 4 is a side partial sectional view of a combination needle filter apparatus and syringe assembly according to another embodiment of the disclosure.
FIG. 5 is a further side partial sectional view of a combination needle filter apparatus and syringe assembly according to the embodiment shown in FIG. 4.
FIG. 6 is a side partial sectional view of a combination needle filter apparatus and syringe assembly according to a further embodiment of the disdosure.
FIG. 7 is a side partial sectional view in partial phantom of a needle filter apparatus with a stepped tapered syringe receiving neck according to another embodiment of the disclosure.
FIG. 8 is a side partial sectional view in partial phantom of a needle filter apparatus with a pre-attached needle according to a still further embodiment of the disclosure.
FIG. 9 is a side partial sectional view in partial phantom of a needle filter apparatus with a pre-attached syringe needle secured to the syringe-receiving neck according to an additional embodiment of the disclosure.

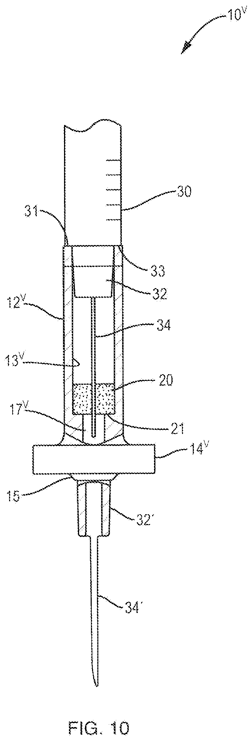

FIG. 10 is a side partial sectional view of a combination needle filter apparatus and syringe assembly according to a still further embodiment of the disclosure.
FIG. 11 is a side partial sectional view in partial phantom of a needle filter apparatus with a pre-filter according to a yet further embodiment of the disclosure.
FIG. 12 is a top perspective view of a modular needle stop segment according to one embodiment of the disclosure.
FIG. 13 is a side sectional view in partial phantom of a needle filter apparatus with an elbow neck according to an alternative embodiment of the disclosure.
FIG. 14 is a side sectional view in partial phantom of a needle filter apparatus with an modified elbow neck according to a further alternative embodiment of the disclosure,
FIG. 15 is a side sectional view in partial phantom of a needle filter apparatus with an elbow neck and elbow inlet according to a still further alternative embodiment of the disclosure.
FIG. 16 is a side elevational view of a needle filter apparatus with a swivel elbow neck and a centrally oriented elbow inlet according to a yet further alternative embodiment of the disclosure.
FIG. 17 is a top elevational view of the needle filter apparatus with an offset swivel elbow neck and a centrally oriented elbow inlet according to the embodiment of the disclosure shown in FIG. 16.
FIG. 18 is a side elevational view of a needle filter apparatus with a 45° elbow neck and centrally oriented elbow inlet according to still another embodiment of the disclosure.
FIG. 19 is a side sectional view of a needle filter apparatus with a hollow fiber membrane in a "U" configuration according to another embodiment of the disclosure.
FIG. 20 is a side sectional view of a needle filter apparatus with a hollow fiber membrane according to yet another embodiment of the disclosure,
FIG. 21 is a side sectional view in partial phantom of a needle filter apparatus with a needle sealing neck according to a further embodiment of the disclosure.
FIG. 22 is a side sectional view of a needle filter apparatus with an extended neck secured to a syringe/needle assembly according to a still further embodiment of the disclosure.
FIG. 23 is a side sectional view of a needle filter apparatus with an extended neck configured to secure to the barrel of a syringe/needle assembly according to a yet further embodiment of the disclosure.
FIG. 24 is a side sectional view of a needle filter apparatus with an extended neck configured with threading to engage the barrel of a syringe/needle assembly according to an alternative embodiment of the disclosure.
FIG. 25 is a side sectional view of a needle filter apparatus with an extended neck configured with threading to engage a threaded hub of a syringe/needle assembly according to a further alternative embodiment of the disclosure.

### DETAILED DESCRIPTION OF THE DISCLOSURE

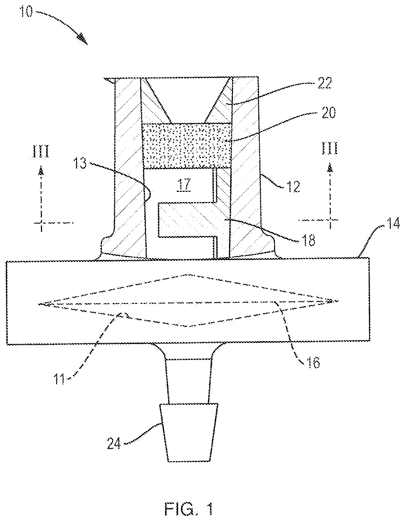

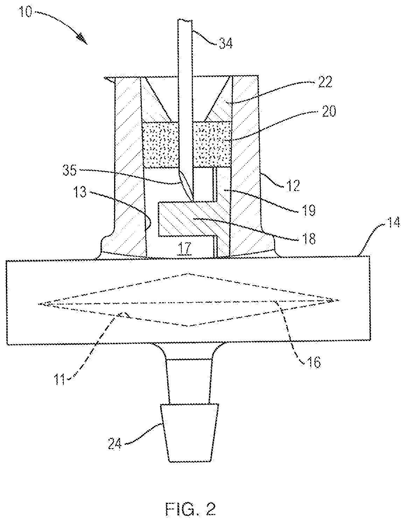

Referring to FIGS. 1 and 2, in one aspect of the disclosure, a truncated needle filter apparatus is shown generally as **10**. Apparatus **10** includes a substantially cylindrical body **14** that defines a generally hollow filter chamber **11** configured to hold one or more filters **16.** Capsule **10** may be formed in other regular or irregular geometric shapes to accommodate a wide variety of filter configurations secured in the capsule. A primary consideration is to use a filter dimensioned to fill filter chamber **11** sufficient to ensure any fluid that enters body **14** will flow through the filter before exiting,

Extending from a first end of body **14** is hose barb **24** configured to receive tubing and the like. The barb can be used directly as an inlet to receive fluid, or may be used as a connecter to receive an end of a tube, the other end of which is placed into a container from which fluid will be drawn. If barb **24** is used without tubing, apparatus **10** is brought to the fluid source so as to submerge the top of barb **24** in the fluid source for aspiration through apparatus **10** and into a temporarily attached syringe and needle assembly.

Extending from a second end of body **14** is truncated syringe receiving neck **12**. Neck **12** is substantially cylindrical in shape and has an inner wail **13** that defines a needle receiving chamber **1:7** in fluid communication with filter chamber **11.** Chamber **17** may be formed in a tapered configuration with the larger diameter end of the taper positioned at an end of neck **12** distal from body **14.** Although truncated neck **12** is configured to receive solely the needle of a needle-bearing syringe, the tapered configuration allows for the barrels of differently sized syringes to engage with inner wall **13** so as to set and stabilize the alignment of the needle with a centerline of chamber **17.** This is particularly so with respect to relatively short needles in the 6 mm range. The apparatus as structured can accommodate conventional needle lengths of from about 6 mm to about 70 mm, but can also be configured and dimensioned to accommodate needle lengths beyond the recited range.

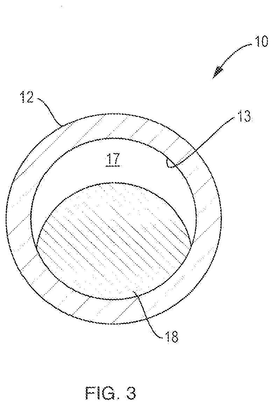

Referring still to FIGS. 1 and 2 and now FIG. 3, extending radially inwardly from inner wall **13** is needle stop segment **18.** Stop segment **18** extends into chamber **17** so as to provide a mechanical barrier to needle travel beyond the shoulder. This ensures any needle inserted into chamber **17,** regardless of the angle of insertion, cannot be advanced into body **14** where it could otherwise pierce filter **16** and compromise the filtering function,

In one embodiment as shown in horizontal cross-section in FIG. 3, stop segment **18** has a radiused profile so as to maximize the cross-sectional area available for fluid flow past the shoulder. This allows for the location of the approximate apex of the radius to cross or pass beyond the centerline of chamber **17.** It should be understood the radius profile can be altered to conform to any geometric shape including a tortuous irregular "over-under" configuration provided the cross-sectional center point of the plane occupied by shoulder **18** in chamber **17** is physically covered by the shoulder. Although the shoulder could be configured to be shorter, such a configuration will increase the possibility of a needle bypassing the shoulder and entering into the filter chamber.

The radially innermost end of shoulder **18** should be extended at least to the chamber centerline, but can be extended further-by way of illustration and not limitation--to align with an inner rim of a seal retention ring, as more fully described below, so as to further minimize the chance of a needle bypassing the shoulder. The more radially extended shoulder **18** is, however, the less area is available for fluid to pass around shoulder **18.** It should be understood the flow path does not have to be large to be effective, but at least should be larger than the inner diameter of the syringe needle. A smaller flow path has the added advantage of reducing the void volume of chamber **17** so as to maximize the fluid recovery from the apparatus. This is particularly relevant and important when the fluid/solution being drawn into the apparatus and the syringe is an expensive drug or medicament.

In integral form, shoulder **18** has portions defining a longitudinal spacer **19** that extends from shoulder **18** in both an upwardly and downwardly direction. A first end extends downwardly from the shoulder and registers against body **14** so as to create a gap between shoulder **18** and the body to allow for fluid to flow from the filter chamber into chamber **17.** A second end of spacer **19** extends upwardly and provides a support surface for a stopper **20** more fully described below. This also creates a gap between shoulder **18** and stopper **20** to allow fluid to flow from the filter chamber into chamber **17** and into a needle secured in truncated neck **12.**

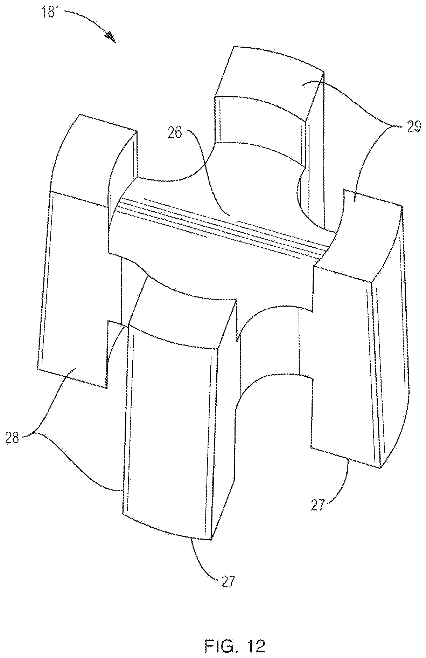

In a modular form as shown illustratively in FIG. 12, stop segment 18 includes a needle strike plate **26** secured between, and continuous with, a plurality of spacer columns **28.** Strike plate **26** is substantially planar in configuration to provide a contact surface for a syringe needle. It should be understood, however, that strike plate **26** does not have to have a planar surface to function as a needle stop. This substantially symmetrical construction about a center point is particularly advantageous to prevent needle passage beyond stop segment **18** regardless of the entry point of a syringe/needle assembly at any point about neck **12.**

Each spacer column **28** is an elongate structure positioned radially outwardly from, and connected to, strike face **26.** Each column **28** has a bottom end **27** configured to register against portions of body **14** so as to form a gap for fluid flow between body **14** and a bottom surface of strike plate **26**. Each column **28** also has a top end **29** configured to provide a registration and support surface for stopper **20.** The combined top ends **29** provide a stable platform to secure support stopper **20** in neck **12** so as to maintain a gap for fluid flow between strike plate **26** and a bottom surface of stopper **20.** Vertical gaps defined between adjacent columns **28** provide fluid passages for fluids/solutions to flow from below strike plate **26** to above the strike plate. A perimeter edge of strike plate **26** formed between the columns may be constructed with inwardly directed radius or scallop profiles to increase the cross-sectional area for fluid flow passed strike plate **26.**

Modular stop segment **18** is dimensioned and configured to have outer walls of columns **28** register against inner wall **13** in a friction fit arrangement. Alternatively, modular stop segment **18** may be secured to the inner wall with adhesives, sonic welding, fusion bond, melting the arranged components to fuse them together, or via the addition of complimentary structural features to create an interference fit as is well known in the art.

Stopper **20** is a silicone or rubber, substantially disc-shaped element dimensioned and configured for insertion into chamber **17.** More specifically, stopper **20** may be constructed from silicon, nitrile, ethylene propylene diene monomer (EPDM), fluoro-elastomers and mixtures thereof. The method of construction may be any conventional method of stopper manufacture well known in the art. There are no special considerations or processes necessary for preparation of stopper **20** for use in apparatus **10** beyond common manufacturing practices.

Stopper **20** is dimensioned to provide a substantially airtight seal when in registration with inner wall **13.** The second end of spacer **19** provides a registration surface for stopper **20** that locates the stopper in the proper spatial orientation relative to shoulder **18** when inserted into chamber **17.** Stopper **20** functions as a seal when a needle **34** is inserted into the stopper. The material of the stopper collapses around the needle after needle penetration. This, combined with the seal formed at the junction of the stopper and inner wall **13** ensures that chamber **17** is substantially airtight to allow for the creation of vacuum pressure in the chamber when the plunger of an attached needle-bearing syringe is retracted to draw fluid through needle filter apparatus **10**into an open end **35** of a needle **34** from a fluid source.

As is common knowledge to one of ordinary skill in the art, the materials used to make stoppers may have a tendency to adhere to inserted needles so much so that the stopper may migrate out of position when an embedded needle is extracted out of the stopper. To ensure stopper **20** is retained in chamber **17** when needle **34** is extracted from apparatus **10,** an annular retainer ring **22** is secured above stopper **20** in registration with a top surface of the stopper. Retainer ring **22** may be integral or modular in design,

If modular, ring **22** may be secured to inner wall **13** via friction fit, interference fit, correspondingly matched threading, adhesive, fusion bonding and the like. If integral, ring **22** may be constructed by melting a top end of neck **12** and forming the retention ring by rolling the melted end into the neck until the melted portion registers against stopper **20.** If constructed in an integral manner, the location of a top surface of stopper **20** and retention ring **22** will be more proximate the distal end of neck **12.** With an integrally formed ring **22,** to achieve the same dimensional gap between a stopper **20** and a stop segment **18** of an apparatus constructed with a modular retention ring, stopper **20** may have to be constructed with a greater thickness (vertical thickness). than one used with a modular retention ring,

An inner wall of ring **22** may have a frustoconical profile in cross section with the narrower diameter end positioned toward stopper **20** and the wider diameter end positioned toward the opening of neck **12.** This configuration creates an annular sloping surface that can be used to redirect needles inserted into neck **12** in a non-orthogonal misaligned orientation toward the desirable entry point at the centerline of stopper **20** and chamber **17** so as to ensure registration against shoulder **18** is achieved if the needle is inserted deep enough.

To use apparatus **10**, a syringe/needle assembly is lowered into truncated neck **12** with the needle tip in the lead position. The syringe should be lowered into the apparatus in an orientation to align the longitudinal axis of the syringe with a centerline of apparatus **10,** It should be understood, however, that the novel configuration, orientation and combination of the truncated neck, sloped retention ring and needle stop permits a user to insert a syringe needle into apparatus **10** at an angle that deviates significantly from the centerline of apparatus **10**-including an initial entry point that deviates significantly from the apparatus centerline-so as to still achieve needle tip registration against stop segment **18** when the needle is fully inserted into the apparatus. Angular deviations of as much as 45° and greater from the apparatus centerline will result in successful joinder of the syringe/needle assembly to the apparatus.

To continue the insertion process, the syringe/needle assembly is advanced into neck **12** so as to pierce stopper **20** with the needle tip. The syringe/needle assembly is advanced until the needle tip passes completely through the stopper. The assembly may be advanced until the needle tip contacts shoulder **18,** but this latter step is not necessary as long as the needle tip has passed beyond stopper **20** into chamber **17.** The combined syringe and apparatus is now ready for fluid aspiration.

To aspirate fluid into the syringe, the syringe/apparatus **10** assembly is lowered into a fluid/solution containing vessel or container so as to submerge the end of inlet port **24** below the surface of the fluid/solution. If any accessory elements are attached to port **24,** e.g., a tube, the end of the tube is submerged into the fluid/solution. Once this positioning is accomplished, the syringe plunger is retracted so as to create a vacuum in chamber **17.** The vacuum is extended into the connected filter chamber and into the inlet port **24** and any attached accessory. This draws the fluid into apparatus **10** through the port, into the filter chamber, through filter **16,** into chamber **17,** into the needle lumen and into the syringe barrel. Once the desired amount of fluid/solution is drawn into the syringe, apparatus **10** is removed from the syringe needle. Apparatus **10** may be discarded or reused depending upon the aseptic conditions of its prior use.

In one embodiment, filter **16** may be constructed as a hydrophilic porous membrane. As fluid flows into apparatus **10** and onto filter **16**, the hydrophilic membrane will be wetted spontaneously and permit the fluid to flow through filter **16** and into the syringe/needle assembly. This construction functions well in normal operation in the absence of air or gas bubbles. When air bubbles enter the apparatus during the fluid aspiration process, the air bubbles will accumulate on the surface of hydrophilic filter **16.** This creates an "air-locking" event in which no further fluid can pass through the wetted hydrophilic membrane until the membrane's bubble point pressure is exceeded to clear the air bubble(s) out and allow fluid: to flow again. To prevent such air-lock events, a filter **16** constructed with one or more hydrophilic and hydrophobic sections may be used. The differing sections may be co-planar or occupy different planes or levels in the filter so long as fluids/gases introduced into the apparatus will have access to both types of filter sections.

In one advantageous embodiment to prevent "air-lock", filter **16** (in the form of--by way of example--an approximately 13mm disc membrane) is constructive primarily of a predominantly hydrophilic membrane with a small section (e.g., a 3mm diameter dot, strip, or even half of the disc membrane) being hydrophobic. As fluid flows into apparatus **10,** the hydrophilic section of filter **16** is wetted while the hydrophobic dot, strip, or section remains dry due to its hydrophobicity. Should air and/or gas enter the apparatus, the air and/or gas will pass through the hydrophobic section of filter **16** so as to prevent an air-lock event in the apparatus. The same filter construction can also function to prevent liquid or "water-locking" events when used to filter gaseous fluid that may contain some water. The presence of the hydrophobic and hydrophilic sections on filter **16** permits both gas/air and liquid/water passage and prevent gas/air or liquid/water- locking events (depending upon the application).

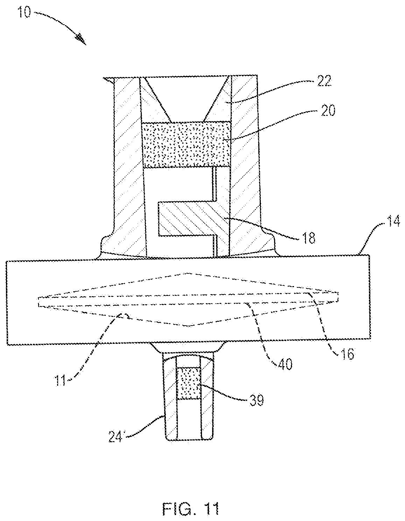

Referring now to FIG. 11, apparatus **10** is shown with an optional pre-fitter **39** secured via ultrasonic welding or similar attachment means in an inlet port **24'** configured in a slip seal configuration. It should be understood the implementation of a pre-filter is not dictated by the inlet port configuration. Other configurations, barb and luer lock configurations as illustrative examples, can be used in connection with a pre-filter. Pre-filter **39** is included to perform gross filtration of relatively large particulate matter on the order of about 30µm to about 200µm in diameter. The mechanisms used to remove relatively large diameter contaminant particles may include non-sieving adsorption, sieving, combination sieving and non-sieving adsorption and the like.

The incorporation of pre-filter **39** (and/or an optional second pre-filter **40** disclosed herein) is used to protect filter **16** from premature plugging by excessive amounts of contaminants that might be in the fluid ultimately drawn through filter **16.** Pre-filter **39** and **40** can be used in combination or alone as disclosed more fully herein. As stated, pre-filter **39** is for very course filtration of particles on the order of about 30-200 microns, whereas pre-filter 40 (in a microporous membrane form) may filter particles on the order of from about 0.2 microns to about 3 microns. This is in contrast to filter **16** that may filter particles on the order of from about 0.01 microns to about 1.2 microns. The difference is particle size filtration is dictated by the respective differences in the membrane pore sizes wherein pre-filter **40** has larger pore sizes than filter **16** as disclosed herein.

Pre-filter **39** may be secured in the apparatus at any point upstream of the main filter **16** including an upstream section of filter chamber **11** Alternatively, or in addition, a pre-filter may also be positioned downstream of filter **16** to perform gross filtration for bi-directional flow configurations. As used herein, "upstream" shall mean the side of main filter **16** opposite the side in which a syringe/needle assembly is inserted. Location in inlet port **24**' is particularly advantageous to prevent large particulate contaminants from travelling beyond tile inlet into the apparatus.

Pre-filter **39** may be constructed from coarse sponge material with pore sizes from about 10 microns to about 200 microns, screen materials such as nylon, polyester with mesh openings from about 20 microns to about 200 microns, or any fibrous filter media formed with micro-glass fibers, nonwoven polymeric microfibers or nanofibers. This ensures only the largest pieces of contaminant matter will be restricted by pre-filter **39**. Depth media constructed from polypropylene is another alternative that may be used advantageously for this purpose. Although a hydrophobic polymer, filter material made from polypropylene allows for the passage of air and liquid due to its low bubble point.

The pre-filter may also be arranged in series with one filter membrane layer registered against one or more additional layers in an orientation substantially perpendicular to the direction of fluid flow through the apparatus. The layers collectively may be hydrophilic, hydrophobic, or a mixture of both. Each layer may be uniformly hydrophilic or hydrophobic, or may include areas modified to have the opposite characteristic. However configured, to ensure air and gas passage through the filter assembly, the hydrophobic section has to be continuous from an upstream surface to a downstream surface of the filter assembly. If hydrophilic layers with modified hydrophobic sections are stacked in series, the hydrophobic sections have to align sufficiently to provide a continuous hydrophobic channel from an upstream to a downstream side of the membrane assembly.

A further optional second pre-filter **40** may be secured (via ultrasonic welding) further downstream from pro-fllter **39** in inlet port **24**' or in filter chamber **11** upstream of filter **16** so as to provide additional pre-filtration. Alternatively, or in addition, a second pre-filter can be secured in the apparatus downstream of filter **16** for bi-directional flow applications. The construction of pre-filter **40** may conform to any of the variants described for filter **16**. The primary difference between pre-filter **39** and second pre-filter **40** (apart from the microporous construction of the pre-filter **40** membrane) is the location of the filters and the retention function in the filtration process.

Pore sizes of each layer of a multilayer membrane assembly, e.g., second pre-filter **40** positioned on the upstream side of filter **16**, may be the same for all the layers, or may differ from one layer to another. For membranes used as pre-filter **40**, the pore sizes may range from about 0.2 micron to about 10 microns, while filter **16** may have pore sizes from about 0.01micron to about 5 micron. In one advantageous embodiment, the: pore sizes of pre-filter **40** are set to be larger than the: pore sizes of filter **16.**

When both pre-filter **40** and fitter **16** are constructed as hydrophilic membranes, air-locking events can occur. To prevent this, hydrophobic layers or sections may be introduced into the construction. The layers collectively may be hydrophilic, hydrophobic, or a mixture of both. Each layer may be uniformly hydrophilic or hydrophobic, or may include areas modified to have the opposite characteristic. However configured, to ensure air and gas passage through the filter assembly, the hydrophobic section has to be continuous from an upstream surface to a downstream surface of the filter assembly. If hydrophilic layers with modified hydrophobic sections are stacked in series, the hydrophobic sections have to align sufficiently to provide a continuous hydrophobic channel from an upstream to a downstream side of the: membrane assembly.

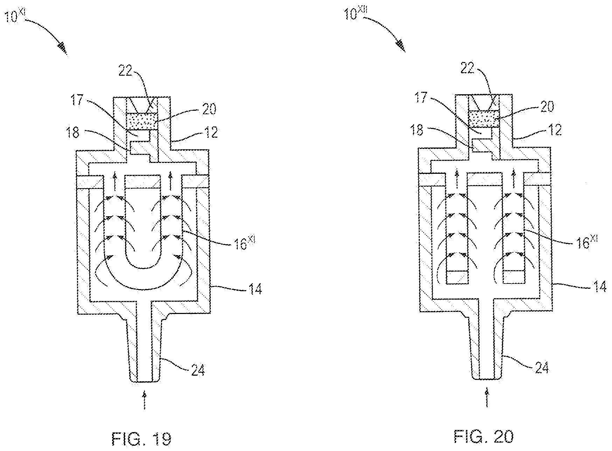

In this embodiment, filter **16** is positioned downstream from one or more pre-filters to perform the final filtration step in which the smallest sized contaminant particles are removed from the aspired fluid/solution. Like the other disclosed filters, filter **16** may be structured from a single layer or multiple membrane layers. The layers may be configured as "flat" sheets, as tubular or hollow fiber membranes, (annular "U" shaped membrane **16^{XI}** in FIG. 19, annular shaped membrane **16^{XII}** in FIG. 20 with annular filter end cap **16a**), or as combinations of the two configurations. In similar manner to the optional pre-filters, to prevent the possibility of gas lock, hydrophobic layers or hydrophilic layers with modified hydrophobic sections are used to ensure air and gas passage through the filter so as not to inhibit fluid/solution flow through the filter.

Although filter **16** can be constructed with the same materials and configurations disclosed for pre-filter **39**, filter **16** differs from any pre-filter present in the apparatus with respect to pore size. Filter **16** is constructed with pore sizes ranging from about 0.01µm to about 5µm. Specific pore sizes of 0.1 µm, 0.2µm, 0.45µm, 0.8µm and 1.2µm are suitable for filter **16.** Like the disclosed pre-filters, it should be equally understood with respect to filter **16** that any filter media or membrane configurations disclosed herein may be configured to permit bi-directional flow.

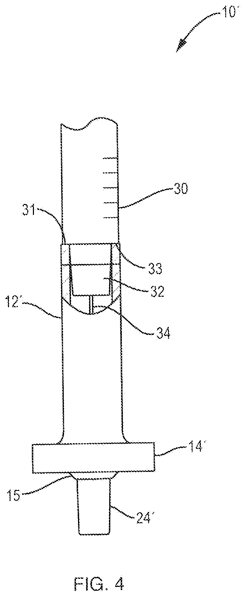

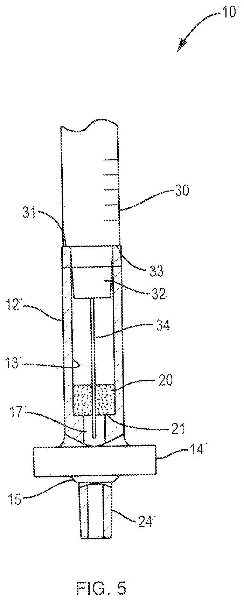

Referring now to FIGS. 4 and 5, in another aspect of the disclosure, a needle filter apparatus shown generally as **10'** includes an extended neck segment to engage the distal port and/or barrel of a syringe/needle assembly secured to the apparatus. It should be understood that elements referenced with primed numbers in one embodiment correspond to elements in other embodiments referenced with the same unprimed or differently primed numbers. This embodiment uses additional contact points to improve needle/apparatus alignment and stability, and to prevent needle penetration of the filter membrane.

The primary components of needle filter apparatus **10** shown in FIGS. 1 and 2 are substantially the same for apparatus **10**'. Apparatus **10**' has a main body **14**' that defines a filter chamber for receiving a filter **16**'. An inlet port **24**' in the form of a slip seal (shown), barb (shown in FIGS. 1 and 2), luer lock and the like extends from a first end of body **14**' has portions defining a port chamber in fluid communication with the filter chamber. Inlet port **24'** may also include an annular ring **15** to improve structural support at the junction of the inlet to body **14**',

Extending from a second end of body **14**' is extended syringe receiving neck **12**'. Neck **12**' is substantially cylindrical in shape and includes an inner wall **13'** that defines a cylindrically shaped needle chamber **17**'. Chamber **17** may be tapered in longitudinal cross-sectional shape with the larger diameter end of the taper positioned at the end of neck **12'** distal from body **14**'. The tapered configuration allows for the neck's distal end to receive distal ports **32** and/or barrels of syringes **30** of various cross-seetional diameters. Larger diameter syringes inserted into the distal end of chamber **17**' slide fess of a distance into the chamber until an outer wall of the syringe distal port **32** and/or barrel wall engages inner wall **13**' in a friction fit connection. Conversely, syringes with smaller cross-sectional diameter barrels can be inserted further into chamber **17'** before registering against and forming a seal with inner wall **13**'. This creates a substantially airtight seal at the plane occupied by the leading segment of the syringe that engages inner wall **13**'.

Although this embodiment requires the cross-sectional dimension of neck **12**' to be customized for differently sized syringes, the extra contact between: the syringe barrel and the extended neck provides additional support: to secure the needle in the apparatus. The registration of the syringe barrel against inner wall **13'** improves and ensures the attached needle is substantially aligned with the centerfine of stopper **20'** substantially orthogonal to the plane occupied by stopper **20'** so as to positively engage shoulder **18**' after penetration of stopper **20'**. This significantly reduces the possibility of bypassing shoulder **18'**.

The: registered surfaces of the neck and syringe port may form a slip seal, friction fit connection, or may be constructed with other elements to create interference fit or luer lock type connections. It is within the spirit and scope of the disclosure to use any conventional method of securing a syringe body to neck **12'**.

Portions of a region of inner waif **13'** proximal to, but separate from body **14'** define an annular stopper support shoulder **21** configured and dimensioned to provide a registration surface for stopper **20.** Stopper **20** is inserted into the open end of neck **12**' and advanced into the neck until in contact with shoulder **21.** The placement of shoulder **21** on inner wall **13**' can be varied to enlarge or reduce chamber **17**'. As should be understood by anyone of ordinary skill in the art, a smaller chamber **17**', by volume, reduces the overall distance aspired fluid has to travel in order to enter into needle **34.**

Stopper **20** is dimensioned to expand into inner wall **13'** to form a substantially airtight seal. A stopper retainer ring **22** may or may not be inserted above stopper **20** to secure the stopper in its position when needle **34** is retracted from the assembly If ring **22** is incorporated into neck **12'**, it does not require the sloped inner annular surface as extended neck **12**' assists and performs the syringe/needle alignment to apparatus **10**'.

In this embodiment, a needle stop shoulder is not required to limit the travel of needle **34** in neck **12**'. In place thereof, a top annular surface **31** of neck **12'** functions as a stop by engaging an annular syringe shoulder **33** formed by the junction of the syringe barrel and syringe port **32.** In an alternative embodiment, a needle stop shoulder can be incorporated into neck **12'** positioned between body **14**' and stopper shoulder **21.** The same criteria and conditions necessary for the construction of shoulder **18** in the truncated neck version apply equally to a corresponding shoulder in extended: neck **12**'.

To use this embodiment, a needle-bearing syringe **30** is inserted into neck **12'** until syringe shoulder **33** registers against neck surface **31.** In the process of lowering syringe **30** into neck **12**', needle **34**should pierce stopper **20** completely through so that needle tip **35** (shown in FIG. 2) is resident in chamber **17**'. This ensures the lumen of needle **34** is in fluid communication: with: chamber **17**', which, in turn, is in fluid communication with the filter chamber and the fluid channel of inlet port **24**'. If needle **34** does not completely penetrate stopper **20** before syringe **30** engages neck shoulder **31,** the proper orientation will not be achieved to generate a vacuum in chamber **17'** to draw fluid into the apparatus and syringe when the syringe plunger is retracted. Accordingly, the version of apparatus **10'** chosen for a specific syringe/needle assembly must be selected not only on the basis of the neck cross-sectional diameter, but also by its length to ensure full penetration of stopper **20** when the syringe is engaged to apparatus **10'**.

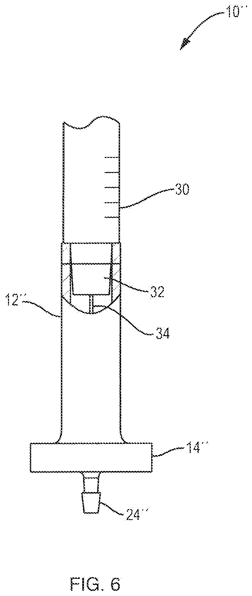

In an alternative embodiment shown in FIG. 6, a needle filter apparatus shown generally as **10"** includes a filter body **14"** that defines a filter chamber for housing a filter. Extending from a second end of apparatus **10**" is an extended neck **12**" configured and dimensioned to receive a syringe and attached needle in similar fashion to the method of attachment to neck **12'** of apparatus **10**'. Neck **12"** shares the same features of neck **12**' as disclosed herein, In contrast to apparatus **10'**, apparatus **10**" has a barb **24"** extending from a first end of filter body **14"** in place of slip seal port **24'** configured to receive tubes and like elements useful for drawing fluids from containers and vessels accessible with an elongate tube. The method of using apparatus **10**" is substantially the same as the method of use for apparatus **10**' with the exception of the means used to access the fluid/solution desired for aspiration.

In a further alternative embodiment shown in FIG, **10**, a needle filter apparatus shown generally as **10^{v}** includes a fitter body **14^{v}** that defines a filter chamber **11^{v}** for housing a filter. Extending from a second end of apparatus **10^{v}** is an extended neck **12^{v}** configured and dimensioned to receive a syringe **30** and attached needle **34** in similar fashion to the method of attachment to neck **12'** of apparatus **10**'. Neck **12^{v}** shares the same features of neck **12**' as disclosed herein. In contrast to apparatus **10**', apparatus **10^{v}** has an accessory needle **34'** secured via an accessory needle hub **32**' to a first end of filter body **14^{v}** in place of slip seal port **24**'.

Accessory needle **34**' is configured to draw fluids from containers such as vials that require the insertion of a needle into a membranous cap, or containers such as ampules that require a relatively narrow neck portion to be snapped off to access the fluid. The method of using apparatus **10^{v}** is substantially the same as the method of use for apparatus **10**' with the exception of the means used to access the fluid/solution desired for aspiration. Like apparatus **10**', apparatus **10^{v}** may be discarded after use. For user convenience, apparatus **10^{v}** may be provided in kit form with a corresponding syringe/needle assembly.

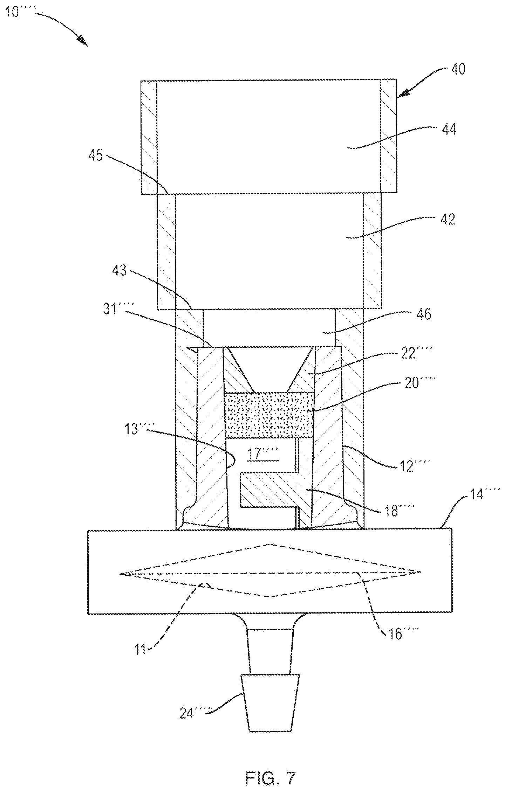

Referring now to FIG. 7, in a yet further aspect of the disclosure, a needle filter apparatus shown generally as **10"**" includes a stepped tapered extended neck **40** configured and dimensioned to accommodate syringes with different cross-sectional diameters and lengths in the embodiment shown in FIG. 7, neck **40** is modular in configuration and fits superposed about truncated neck **12**"". Alternatively, neck **40** may be made an integral part of the apparatus during the molding process. The combination of stepped neck **40** and truncated neck **12**"" functions as a hybrid of apparatus **10** and apparatus **10**' by providing additional contact surfaces for alignment of a syringe to the apparatus.

Apparatus **10**'"' has elements substantially identical to the elements of apparatus **10,** A filter body **14**"" has portions defining a filter chamber for housing a filter **16**"". An inlet **24**"" extends from a first end of body **14**"" and defines a channel in fluid communication with the filter chamber. A truncated neck **12""** extends from a second end of body **14**"" and has an inner wall **13**"" that defines a chamber **17**"". Neck **12**"" encloses a stopper **20**"" in similar manner to neck **12** and may, or may not be formed with a needle stop shoulder **18**"". The embodiment shown includes shoulder **18**"", It should be understood that the incorporation of neck **40** eliminates the need for shoulder **18**"" as explained below.

Neck **40** includes a series of consecutive annular tapered segments **42** and **44** each having a larger cross-sectional diameter than a preceding segment The tapers are longitudinal in cross-section. The junction of the segments form annular shoulders, **43** and **45** configured and dimensioned to function as stops to limit the intrusion distance travelled by a syringe/needle assembly inserted into the apparatus. A first tapered segment **46** located the most proximal of all the segments to body **14**"" may use annular surface **31**"" of neck **12**"" as its annular shoulder for limiting syringe insertion. As should be understood from a review of FIG. 7, the larger the syringe, the more distal the segment engaged by the syringe assembly will be from body **14**"". By including additional contact surfaces for syringe engagement, alignment and control of the syringe in relation to the apparatus is improved.

As with the other embodiments and aspects of the needle fitter apparatus, apparatus **10**"" is intended to provide a means to filter a fluid/solution for aspiration into a syringe after which the apparatus may be discarded. The method of using apparatus **10**"" is substantially identical to the methods of use for apparatuses **10** and **10'** as disclosed herein.

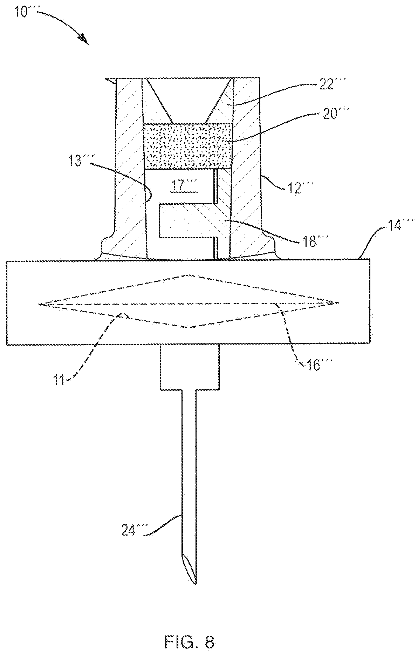

Referring now to FIG. 8, a yet further embodiment of the needle filter apparatus is shown generally as **10**"' This embodiment is substantially identical to needle fitter apparatus **10** in construction and use. The exception is the substitution of a needle **24**"' for inlet port **24.** Needle **24**"' is secured to filter body **14**'" via hub **25** that may be structured with luer lock features, slip seal configuration features, secured via adhesive and the like. Hub **25** may also be an integral part of the apparatus incorporated in the molding process. Apparatus **10**"' may also be provided with a needle cover (not shown) for apparatus needle **24**'" for use before and after apparatus **10**"' has been used.

This embodiment is particularly useful when the fluid/solution to be filtered is contained in a sealed ampule or like container that requires penetration of a sealing membrane or cap. Like with apparatus **10,** a syringe/needle configuration is secured to apparatus **10**"' in like manner used for apparatus **10** and the combined assembly is maneuvered to insert apparatus needle **24**'" into the desired fluid/solution container so as to submerge the needle's tip below the fluid surface. The process for drawing out the fluid is the same as that for apparatus **10.** Apparatus **10**'" may be discarded after use.

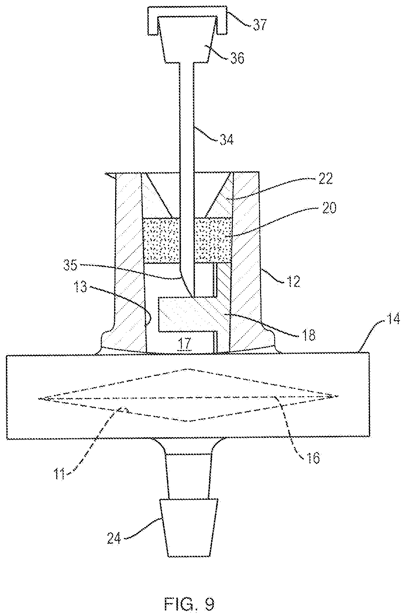

In a yet further aspect of the disclosure as shown in FIG. 9, a needle filter apparatus shown generally as **10** may include a pre-attached syringe needle assembly shown generally as **34.** Needle **34 is** secured to apparatus **10** by piercing stopper **20** with the needle and advancing the needle until needle tip **35** passes completely through stopper **20.** Needle **34** may or may not be advanced until registration with shoulder **18**. If pre-attached, an optional needle-hub cap **37** may be secured to a hub **36** of needle **34.** The means used to secure cap **37** to hub **36** may include luer lock and slip seal configuration features as is well known in the art.

The materials used to construct shoulder **18** should have sufficient resiliency to prevent penetration of a hypodermic needle. This ensures needle tip **35** and the needle lumen remain patent throughout the aspiration and filtering process.

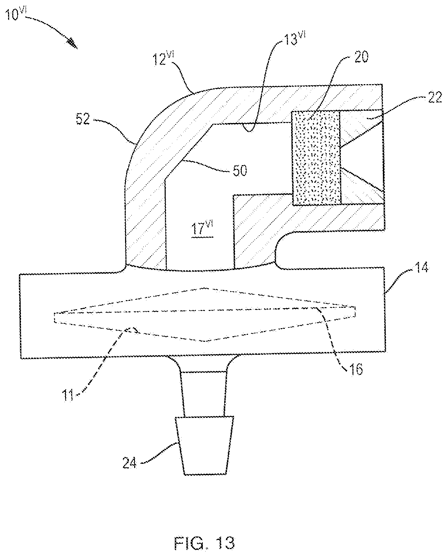

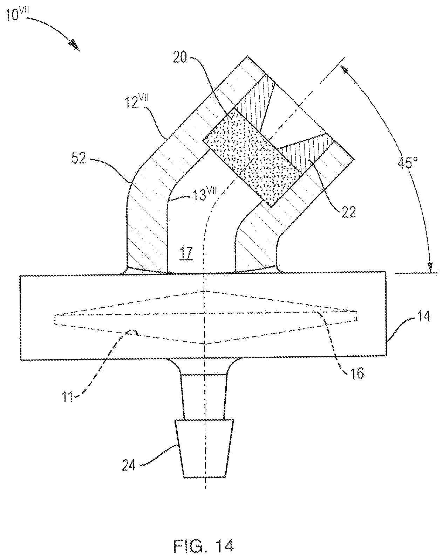

In a still further aspect of the disclosure shown in FIGS. 13-18, alternative embodiments of the needle filter apparatus have constructions that eliminate the need for needle stop shoulder **18.** Referring now to FIG. 13, a needle filter apparatus shown generally as **10^{vi}** includes the same features as apparatus **10** except for the exclusion of needle stop shoulder **18.** In place of shoulder **18,** neck **12^{vi}** is formed with an elbow **52** to change the angle or orientation of the neck opening relative to body **14**. In FIG. 13, the distal portion of the neck is offset approximately 90° from the axis perpendicular to the plane occupied by filter **16.** The offset angle can be varied significantly without compromising the function of the elbow configuration. FIG. 14 shows an alternate embodiment with the distal portion of neck **12^{vii}** offset approximately 45°.

Stopper **20** is secured in the distal portion of neck **12^{vi}** (or neck **12^{vii}** in FIG. 14) downstream from elbow **52.** The change in angular orientation causes any syringe needle inserted into the neck and stopper **20** to register against an elbow inner wall **50** in chamber **17^{vi}** (or **17^{vii}** in FIG. 14). Wall **50** may be configured as a planar surface (shown), as a radiused surface that follows the general shape and contour of the elbow portion of neck **12^{vi}**, or as a non-planar surface. Regardless of the angle or orientation of insertion, any needle introduced into apparatus **10^{vi}** (or **10^{vii}**) will not be able to reach, and therefore, not be able to penetrate filter **16** due to the geometries of the apparatus elements. Because of these geometries, the need for stop shoulder **18** is eliminated.

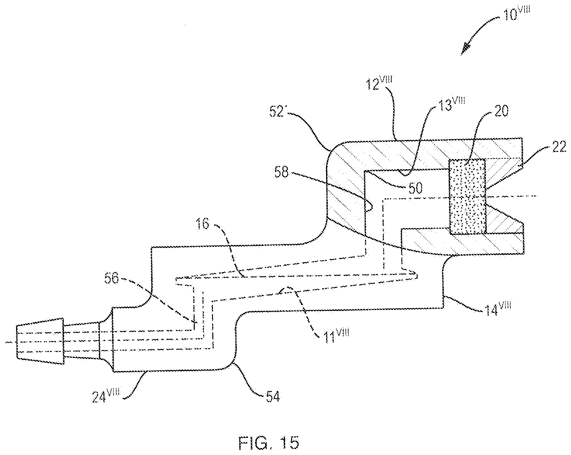

Referring now to FIG. 15, a yet further aspect of the disclosure is shown with a modified neck and modified inlet. In this embodiment in which the apparatus is shown generally as **10^{viii}**, neck **12^{viii}** originates from an end of body **14^{viii}** rather than from an approximate center section as shown in prior embodiments. Neck **12^{viii}** is formed with essentially the same elbow **52** disclosed in FIG. **13** except inner elbow wall **52'** is configured as a substantially sharp approximately 90° corner. This configuration results in the formation of an inner neck back wall **58** positioned in direct alignment with a central axis of stopper **20,** This geometric configuration causes any syringe needle inserted into the neck and stopper **20** to register against back wall **58.** Regardless of the angle or orientation of insertion, any needle introduced into apparatus **10^{viii}** will not be able to reach, and therefore, not be able to penetrate filter **16** due to the geometries of the apparatus elements.

An additional alteration of this embodiment is seen in the walls of filter chamber **11^{viii}**. To promote better flow of fluid through the chamber, the walls of chamber **11^{viii}** are tilted relative to the plane occupied by body **14^{viii}**. This promotes the up flow of any air or gas that enters into the chamber and increases the area between filter **16** and the junctions of the chamber with inlet port **24^{viii}** and with neck **12^{viii}**.

In similar fashion to neck **12^{viii}**, inlet port **24^{viii}** originates from an end of body **14^{viii}** opposite the end from which neck **12^{viii}** originates. Like neck **12^{viii}**, inlet **24^{viii}** is formed with an inlet elbow **54** and a corresponding channel elbow **56** in fluid communication with the entry segment of the inlet channel and the segment connected to, and in fluid communication with, chamber **11^{viii}**. In this embodiment, both the neck elbow and the inlet elbow are configured to be approximately 90°, but in opposite directions so as to create entry and exit points set approximately 180° apart, albeit with a stepped interval in the form of body **14^{viii}**, This configuration allows for the aspiration of fluids from containers or vessels requiring lateral access.

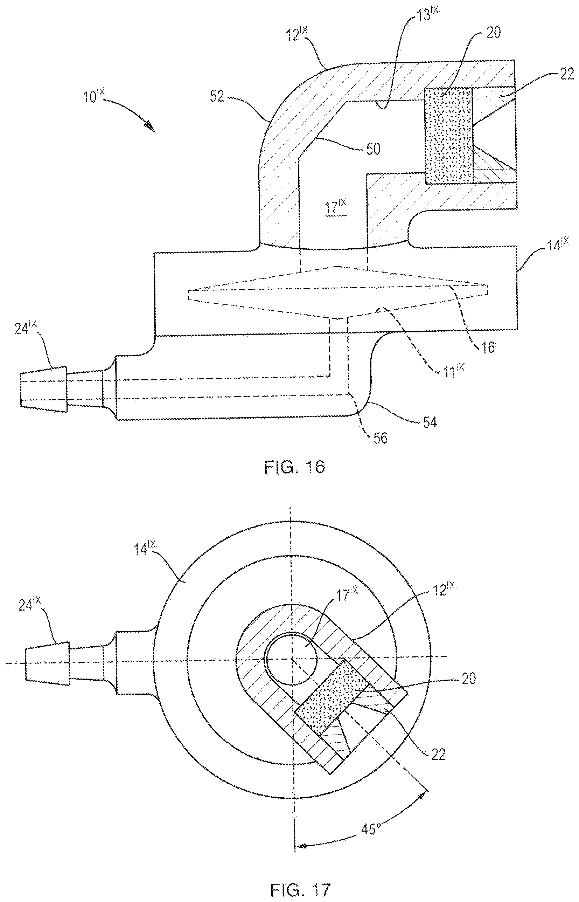

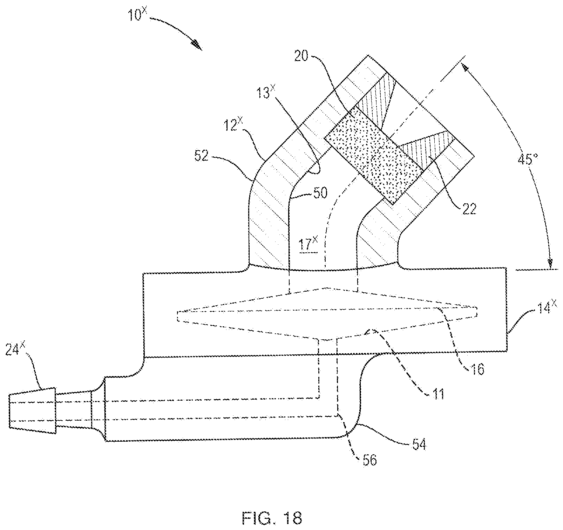

Referring now to FIGS. 16 and 17, in a yet further alternative aspect of the disclosure, a needle filter apparatus shown generally as **10^{IX}** includes a swivel elbow neck **12^{IX}**. In this embodiment, neck **12^{IX}** originates substantially in alignment with a center of body **14^{IX}**. Body **14^{IX}** and a proximal end of neck **12^{IX}** are formed with annular interlocking surfaces that permit the substantially free rotation of neck **12^{IX}** about a central axis of body **14^{IX}**. Sealing elements, e.g., o-ring, etc. are used to create a substantially air tight seal at the body/neck junction. This adds an extra degree of freedom to orient the apparatus for specific applications by swiveling neck **12^{IX}** to a desired orientation relative to the static orientation of inlet **24^{IX}**. FIGS. 16 and 17 show the neck elbow with a 90° bend. FIG. 18 shows the same swivel elbow with a 45° bend. It should be understood the elbow angle can be modified from these illustrative angles without departing from the spirit and scope of the disclosure. In like fashion, the 90° angle of elbow **54** of inlet **24^{IX}** can be modified in a similar manner.

Neck **12^{IX}** (or **12^{X}** in FIG. 18) is configured substantially the same as the neck of apparatus **10^{vi}** shown in FIG. 13. The elbow formed in the neck replaces shoulder **18** as a registration/stop surface for a syringe needle.

inlet port **24^{IX}** (or **24^{X}** in FIG. 18) is configured substantially the same as the inlet port of apparatus **10^{viii}** shown in FIG. 15 except the point of origin is shifted to a central section of body **14^{IX}** (or **14^{X}** in FIG. 18). The configuration allows for the same lateral access to fluid-containing vessels.

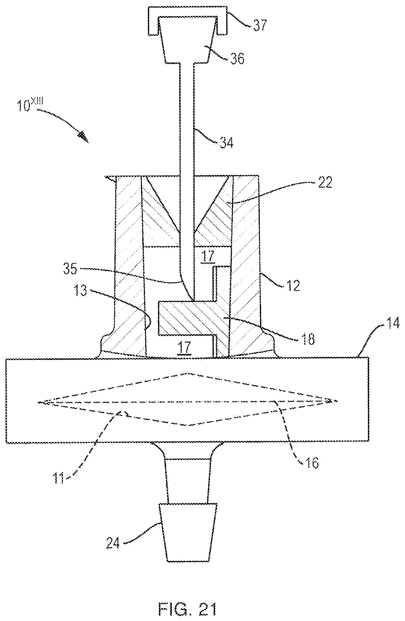

In a still further aspect of the disclosure in which stopper **20** is eliminated, neck configurations permit the formation of a direct seaf with either a syringe needle, a syringe hub, a syringe barrel, and/or combinations of the three. Referring now to FIG. 21, needle filter apparatus shown generally as **10^{XIII}** is configured substantially the same as apparatus **10** with the exception that retainer ring **22^{XIII}** is dimensioned and configured to receive syringe needle **34** so as to create a seal between the outer wall of the needle and an annular inner wall of ring **22^{XIII}**. The tight tolerance of the corresponding dimensions provides a sufficient seal to allow for the generation of a vacuum in neck chamber **17^{XIII}** without the presence of stopper **20.** Ring **22^{XIII}** should be constructed from a material with sufficient inherent lubricity to allow for the insertion of needle **34.** Alternatively, a lubricant can be used to ease needle insertion.

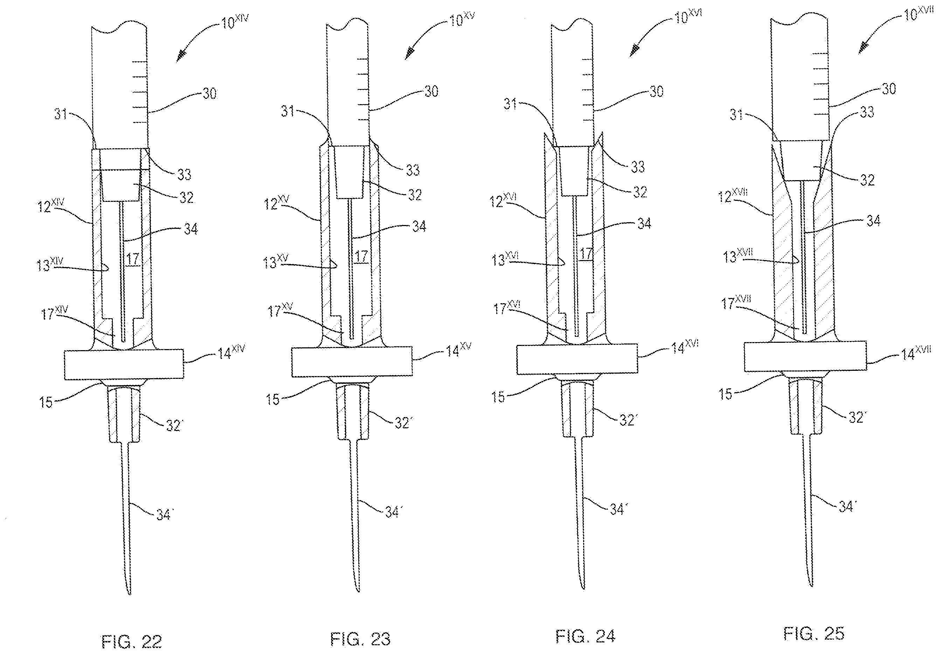

Referring now to FIG. 22, an alternate embodiment of the needle filter apparatus is shown generally as **10^{XIV}** in which the cross-sectionat diameter of neck inner wall **13^{XIV}** is dimensioned to create a friction fit (press fit) with syringe hub **32.** FIG. 23 shows a similar configuration in which the cross-sectional diameter of neck inner wall **13^{XV}** is dimensioned to create a friction fit with an outer wall of syringe barrel **30.** These configurations eliminate the need for stopper **20** in order to create the vacuum in neck chamber **17^{XIV}** (or chamber **17^{XV}** in FIG. 23) necessary to effectuate fluid aspiration into the apparatus and the adjoined syringe/needle assembly.

Referring now to FIGS. 24 and 25, additional alternate embodiments of the needle filter apparatus are shown that incorporate threaded segments to secure a correspondingly threaded syringe. In FIG. 24, the apparatus shown generally as **10^{XVI}** has a distal end of inner wall **13^{XVI}** formed with threading to engage corresponding threading on syringe hub **32.** In FIG. 25, the apparatus shown generally as **10^{XVII}** has a distal end of inner wall **13^{XVII}** formed with threading to engage corresponding threading on syringe barrel **30.** These: configurations also eliminate the need for stopper **20** in order to create the vacuum in neck chamber **17^{XVI}** in FIG. 24 (or **17****^{XVII}** in FIG. 25) necessary to effectuate fluid aspiration into the apparatus and the adjoined syringe/needle assembly.

It should be understood the configurations disclosed to secure the syringe/needle assembly to the apparatus are illustrative in purpose and should not be considered as limiting in scope. Additional alternatives for connecting a syringe to the apparatus so as to create a seal include, but are not limited to, interference fit features such as corresponding luer lock features (male luer configuration on one, female configuration on the other), and slip seals such as o-rings. Any known means for securing a syringe to the apparatus should be considered within the scope of the disclosure.

The filters used in any of the disclosed embodiments of the needle filter apparatus may be constructed from hydrophobic, hydrophilic or hybrid combination hydrophobic/hydrophilic materials. One or multiple layers or membranes with the same or different hydrophobicity and/or hydrophilicity properties may be used to filter the desired fluid/solution. The use of both hydrophilic and hydrophobic filter media is particularly advantageous in certain applications that involve the presence of both aqueous liquids and gases. The hydrophilic media permits the passage of the fluid component through the filter white the hydrophobic media permits the passage of the gaseous component through the filter. This assures flow will not be interrupted or blocked due to the specific characteristics of the fluid/gas mixture.

Multiple layers can also be implemented with any of the disclosed apparatus embodiments to perform different tasks such as pre-filtration or other filtration process enhancements, e.g., removal by non-sieving adsorption, combined sieving and non-sieving mechanisms, ionic charges, etc: Hydrophilic and hydrophobic media and/or membrane types can be used exclusively or in combination to achieve the desired pre-filtration and/or other enhanced filtration objectives that fall within the spirit and scope of the disclosure. A pre-filter media can also be implemented at the inlet or outlet fitting to the filter. Any media type including very coarse sponge or screen pre-filter versions can be used for the purpose of pre-filtration or other enhancements of the filtration process.

Filter materials that exhibit spontaneously wettable properties are also advantageous to the disclosure. Spontaneously wettable material is particularly suitable for the filter apparatus as it allows for the substantially spontaneous wetting out of the filter material to promote and maximize fluid flow through the media.

Each filter media is constructed from fibrous material, including, but not limited to, microfibers and nanofibers of polyethylene, polypropylene, nylon, polyester, carbon, fiberglass, polypropylene sulfide (PPS), Polytetrafluoro-ethylene (Teflon® PTFE), polyvinylidene fluoride (PVDF), polyacrylonitrile (PAN), Ethylene chlorotrifluoroethylene (ECTFE), polyethylene/ultra-high molecular weight polyethylene (PE/UPE) including cellulose/diatomaceous earth or silica blends, cellulose/carbon particles or fibers, cellulose/ion exchange resins, cellulose acetate, nitrocellulose as are available from general media suppliers, as well as combinations of any of the disclosed filter media materials.

Still further filter materials may include microporous, hydrophilic or hydrophobic membranes, including, but not limited to, materials such as polyethersulfone, polysulfone, cellulose acetate, polyvinylidene fluoride: (PVDF), and other fluoropolymers such as perfluoroalkoxy (PFA) and its derivatives, MFA (co-polymer of tetrafluoroethylene and perfluoromethyl vinyl ether and sold under the name Hyflon®), fluorinated ethylene propylene polymer (FEP) and the like, as well as combinations of any of the disclosed fitter media materials.

The media may be constructed from a number of manufacturing processes including, but not limited to, wet-laid processes (similar to papermaking), wet casting, melt-cast, or dry processes such as air-laid, melt-blown, spun-bond, bi-directional starching, etc as is well known in the art.

The apparatus described herein may be constructed from any Injection-moldable thermal plastics, such as polypropylene, polyethylene, high-density polyethylene, nylon, perfluoroalkoxy (PFA), polyvinylchloride (PVC), acrylonitrile butadiene styrene (ABS), polyoxymethylene (POM), and the like. The materials are used in conventional injection molding processes to create the apparatuses in mold halves. The filter material is sonically welded into one half and the two halves are joined together in a final molding step using conventional molding practices well known in the art. A key consideration for material selection, particularly with respect to the needle stop, is to select a material with sufficient resiliency to prevent penetration from sharp metallic objects such as needles

While the present disclosure has been described in connection with several embodiments thereof, it will be apparent to those skilled in the art that many changes and modifications may be made without departing from the scope of the appended claims.

## Claims

1. A needle filter apparatus (10) for filtering fluids and solutions while being aspirated into a syringe (30) for subsequent administration to a patient, comprising:
a filter housing (14) having a first end and a second end and having portions defining a filter chamber (11);
a filter media (16) secured in the filter chamber;
an inlet port (24) extending from the filter housing wherein the inlet port has portions defining a channel in fluid communication with the filter chamber;
a syringe-receiving neck (12) extending from the second end of the filter housing, wherein the neck has portions defining a neck chamber (17) in fluid communication with the filter chamber; the needle filter apparatus being **characterised by**: a needle stop shoulder (18) secured to, or formed in, the neck chamber, wherein the shoulder extends radially inwardly from the neck inner wall (13) so as to intersect a centerline of the neck chamber and to provide a mechanical barrier to needle travel beyond the shoulder, and wherein the shoulder has a first end that extends downwardly toward, and in proximity to, the filter housing so as to create a gap between the shoulder and the filter housing, and a second end that extends upwardly along the neck inner wall to form a stopper registration surface; and,
a needle-sealing stopper (20) secured in the receiving neck, wherein the stopper is configured to register against the stopper registration surface.

2. The needle filter apparatus of claim 1 further comprising a stopper retention ring (22) secured in the syringe-receiving neck, wherein the ring further comprises an inner wall having a frustoconical shape in cross-section, and wherein the ring registers against the stopper and prevents stopper migration or removal from the neck.

3. A needle filter apparatus (10) for filtering fluids and solutions while being aspirated into a syringe (30) for subsequent administration to a patient, comprising:
a filter housing (14) having a first end and a second end and having portions defining a filter chamber (11);
a filter media (16) secured in the filter chamber; the needle filter apparatus being **characterised by**:
an inlet port (24) extending from the filter housing wherein the inlet port has portions defining a channel in fluid communication with the filter chamber;
a syringe-receiving neck (12) extending from the second end of the filter housing, wherein the neck has an inner wall (13) defining a neck chamber (17) in fluid communication with the filter chamber;
a needle stop shoulder (18) secured to, or formed in, the neck chamber, wherein the needle stop comprises a needle strike plate (26) suspended substantially centrally within the neck chamber between a plurality of spacer columns (28) and functioning as a needle stop for a syringe needle (34) of the syringe, wherein the spacer columns are secured to, or integral with, the neck inner wall, wherein each of the plurality of spacer columns comprise a bottom end (27) extending downwardly from the strike plate that collectively form a registration surface for registration against portions of the filter housing, and a top end (29) extending upwardly from the strike plate that collectively form a registration surface for a needle-sealing stopper (20), wherein the bottom ends collectively create a gap between the filter housing and a bottom of the strike plate, and the top ends collectively create a gap between the needle-sealing stopper and a top surface of the strike plate, and wherein the substantially central location of the strike plate prevents a needle from passing beyond the needle stop regardless of the angle or orientation of needle insertion into the apparatus; and,
the needle-sealing stopper secured in the syringe-receiving neck.

4. The needle filter apparatus of claim 3 wherein the strike plate comprises a plurality of inwardly scalloped perimeter regions formed between adjacent spacer columns of the plurality of spacer columns.

5. The needle filter apparatus of claim 1 or 3 further comprising a pre-filter (39) secured in the inlet port upstream of the filter media, wherein the pre-filter has a plurality of pores ranging in size from about 10 µm to about 200 µm, wherein the pre-filter is selected from the group consisting of coarse sponges, screens, membranes having one or more layers, flat membranes, tubular or hollow fiber membranes and mixtures thereof, and wherein the pre-filter is constructed from fibrous material including microfiber and nano-fiber material selected from the group consisting of polyethylene, polypropylene, nylon, polyester, carbon, fiberglass, polypropylene sulfide (PPS), Polytetrafluoro-ethylene (Teflon® PTFE), polyvinylidene fluoride (PVDF), polyacrylonitrile (PAN), Ethylene chlorotrifluoroethylene (ECTFE), polyethylene/ultrahigh molecular weight polyethylene (PE/UPE), cellulose/diatomaceous earth or silica blends, cellulose/carbon particles or fibers, cellulose/ion exchange resins, cellulose acetate, nitrocellulose, polyethersulfone, polysulfone, cellulose acetate, polyvinylidene fluoride (PVDF), and other fluoropolymers such as perfluoroalkoxy (PFA) and its derivatives, MFA (co-polymer of tetrafluoroethylene and perfluoromethyl vinyl ether), fluorinated ethylene propylene polymer (FEP) and combinations thereof.

6. The needle filter apparatus of claim 5 further comprising
a second pre-filter (40) secured in the inlet port or filter chamber [071] upstream of the filter media, wherein the second pre-filter has one or a plurality of layers with each layer having a plurality of pores ranging in size from about 0.2 microns to about 5 microns, wherein the second pre-filter is constructed from fibrous material including microfiber and nano-fiber material and/or porous membrane selected from the group consisting of polyethylene, polypropylene, nylon, polyester, carbon, fiberglass, polypropylene sulfide (PPS), Polytetrafluoro-ethylene (Teflon® PTFE), polyvinylidene fluoride (PVDF), polyacrylonitrile (PAN), Ethylene chlorotrifluoroethylene (ECTFE), polyethylene/ultra-high molecular weight polyethylene (PE/UPE), cellulose/diatomaceous earth or silica blends, cellulose/carbon particles or fibers, cellulose/ion exchange resins, cellulose acetate, nitrocellulose, polyethersulfone, polysulfone, cellulose acetate, polyvinylidene fluoride (PVDF), and other fluoropolymers such as perfluoroalkoxy (PFA) and its derivatives, MFA (co-polymer of tetrafluoroethylene and perfluoromethyl vinyl ether), fluorinated ethylene propylene polymer (FEP) and combinations thereof, and wherein the second pre-filter is selected from the group consisting of coarse sponges, screens, membranes having one or more layers, flat membranes, tubular or hollow fiber membranes and mixtures thereof.

7. The needle filter apparatus of claim 1 or 3 wherein the filter media is a single layer having at least one hydrophilic section and at least one hydrophobic section, wherein each section extends from an upstream surface to a downstream surface of the filter.

8. The needle filter apparatus of claim 1 or 3 wherein the filter media is selected from the group consisting of membranes having one or more layers, flat membranes, tubular or hollow fiber membranes and mixtures thereof, and wherein the filter media has a plurality of pores ranging in size from about 0.01µm to about 5µm.

9. The needle filter apparatus of claim 1 or 3 wherein the filter media comprises a plurality of membrane layers, wherein the layers are all hydrophilic, hydrophobic or mixtures of hydrophilic and hydrophobic.

10. The needle filter apparatus of claim 9 wherein at least one layer of the plurality of membrane layers is either hydrophilic or hydrophobic and wherein the at least one layer has at least one section modified to have the opposite characteristic.

11. The needle filter apparatus of claim 1 or 3 further comprising an extension neck (40) superposed about the syringe-receiving neck, wherein the extension neck is substantially hollow with stepped tapered segments (42, 44) of serially larger cross-sectional diameters.

12. The needle filter apparatus of claim 1 or 3 further comprising a needle (34) secured in the apparatus and a needle-hub cap (37) secured to a hub end (36) of the needle.

13. The needle filter apparatus of claim 1 or 3 wherein the filter media is selected from the group consisting of hydrophilic, hydrophobic and combinations thereof, secured in the filter chamber,
wherein the syringe-receiving neck is an integral or modular extended syringe-receiving neck extending from the second end of the filter housing, and wherein the neck has a top annular surface for registration against a syringe barrel.

14. The needle filter apparatus of claim 13 wherein an outer wall of the inlet port is configured as a barb or as a slip seal surface.

15. The needle filter apparatus of claim 14 further comprising a syringe needle secured to the inlet port.

## Patentansprüche

1. Nadelfiltervorrichtung (10) zum Filtern von Fluiden und Lösungen, während diese in eine Spritze (30) gesaugt werden, für die anschließende Verabreichung an einen Patienten, welche Folgendes umfasst:
ein Filtergehäuse (14), das ein erstes Ende und ein zweites Ende aufweist und das Abschnitte aufweist, die eine Filterkammer (11) bestimmen;
ein in der Filterkammer befestigtes Filtermedium (16);
eine Einlassöffnung (24), die sich von dem Filtergehäuse erstreckt, wobei die Einlassöffnung Abschnitte aufweist, die einen Kanal in fluidem Austausch mit der Filterkammer bestimmen;
einen Hals (12), der die Spritze aufnimmt und der sich von dem zweiten Ende des Filtergehäuses erstreckt, wobei der Hals Abschnitte aufweist, die eine Halskammer (17) in fluidem Austausch mit der Filterkammer bestimmen;
wobei die Filtervorrichtung **gekennzeichnet ist durch**:
eine Nadelstoppschulter (18), die in der Halskammer befestigt oder in ihr ausgebildet ist, wobei die Schulter sich von der Halsinnenwand (13) radial nach innen erstreckt, um eine Mittellinie der Halskammer zu schneiden und eine mechanische Barriere für den Nadellauf jenseits der Schulter vorzusehen, und wobei die Schulter ein erstes Ende aufweist, das sich nach unten hin und in die Nähe von dem Filtergehäuse erstreckt, um eine Lücke zwischen der Schulter und dem Filtergehäuse zu schaffen, und ein zweites Ende, das sich aufwärts entlang der Halsinnenwand erstreckt, um eine Stopperanschlagsfläche auszubilden; und
einen Nadelverschlussstopper (20), der an dem aufnehmenden Hals befestigt ist, wobei der Stopper ausgestaltet ist, sich an der Stopperanschlagsfläche zu anzulegen.

2. Nadelfiltervorrichtung nach Anspruch 1, die ferner einen Stopperrückhaltering (22) umfasst, der an dem Hals, der die Spritze aufnimmt, befestigt ist, wobei der Ring ferner eine Innenwand umfasst, die im Querschnitt eine kegelstumpfförmige Form aufweist, und wobei der Ring sich an dem Stopper anlegt und Stoppermigration oder Entfernung vom Hals verhindert.

3. Nadelfiltervorrichtung (10) zum Filtern von Fluiden und Lösungen, während diese in eine Spritze (30) gesaugt werden, zur anschließenden Verabreichung an einen Patienten, welche Folgendes umfasst:
ein Filtergehäuse (14), das ein erstes Ende und ein zweites Ende aufweist und das Abschnitte aufweist, die eine Filterkammer (11) bestimmen;
ein in der Filterkammer befestigtes Filtermedium (16);
wobei die Filtervorrichtung **gekennzeichnet ist durch**:
eine Einlassöffnung (24), die sich von dem Filtergehäuse erstreckt, wobei die Einlassöffnung Abschnitte aufweist, die einen Kanal in fluidem Austausch mit der Filterkammer bestimmen;
einen Hals (12), der die Spritze aufnimmt und der sich von dem zweiten Ende des Filtergehäuses erstreckt, wobei der Hals eine Innenwand (13) aufweist, die eine Halskammer (17) in fluidem Austausch mit der Filterkammer bestimmt;
eine Nadelstoppschulter (18), die in der Halskammer befestigt oder darin ausgebildet ist, wobei der Nadelstopp ein Nadelschließblech (26) umfasst, das im Wesentlichen zentral in der Halskammer zwischen einer Vielzahl von Abstandshaltersäulen (28) aufgehängt ist und das als Nadelstopp für eine Spritzennadel (34) der Spritze dient, wobei die Abstandshaltersäulen befestigt sind an oder integriert sind in der Halsinnenwand, wobei jede der Vielzahl von Abstandshaltersäulen ein unteres Ende (27) umfasst, das sich von dem Schließblech nach unten erstreckt, was gemeinsam eine Anschlagsfläche für das Anlegen an Abschnitten des Filtergehäuses ausbildet, und ein oberes Ende (29), das sich von dem Schließblech nach oben erstreckt, was gemeinsam eine Anschlagsfläche für einen Nadelverschlussstopper (20) ausbildet, wobei die unteren Enden gemeinsam eine Lücke zwischen dem Filtergehäuse und einer Unterseite des Schließblechs erzeugen, und wobei die oberen Enden gemeinsam eine Lücke zwischen dem Nadelverschlussstopper und einer oberen Fläche des Schließblechs erzeugen, und wobei die im Wesentlichen zentrale Lage des Schließblechs verhindert, dass eine Nadel über den Nadelstopp hinausgeht, ungeachtet des Winkels oder der Richtung des Nadeleinstichs in die Vorrichtung; und
einen Nadelverschlussstopper, der an dem Hals, der die Nadel aufnimmt, befestigt ist.

4. Nadelfiltervorrichtung nach Anspruch 3, wobei das Schließblech eine Vielzahl nach innen rundgezackter Randregionen umfasst, die zwischen nebeneinanderliegenden Abstandshaltersäulen der Vielzahl an Abstandshaltersäulen ausgebildet sind.

5. Nadelfiltervorrichtung nach Anspruch 1 oder 3, die ferner einen Vorfilter (39) umfasst, der dem Filtermedium vorgelagert in der Einlassöffnung befestigt ist, wobei der Vorfilter eine Vielzahl von Poren aufweist, die in der Größe zwischen ungefähr 10 µm und ungefähr 200 µm liegen, wobei der Vorfilter ausgewählt wird aus der Gruppe, die besteht aus grobporigen Schwämmen, Sieben und Membranen, die eine oder mehrere Schichten, Flachmembranen, tubuläre oder Hohlfasermembranen und Mischungen davon aufweisen, und wobei der Vorfilter aus Fasermaterial gestaltet ist, umfassend Mikrofaser- und Nanofasermaterial, ausgewählt aus der Gruppe, die besteht aus Polyethylen, Polypropylen, Nylon, Polyester, Kohlenstoff, Glasfaser, Polypropylensulfid (PPS), Polytetrafluorethylen (Teflon®, PTFE), Polyvinylidenfluorid (PVDF), Polyacrylnitril (PAN), Ethylen-Chlortrifluorethylen (ECTFE), Polyethylen/ultrahochmolekularem Polyethylen (PE/UPE), Zellulose/Kieselgur oder Kieselerdemischungen, Zellulose/Kohlenstoffpartikeln oder -fasern, Zellulose/Ionenaustauschharze, Zellulose-Acetat. Nitrozellulose, Polyethersulfon, Polysulfon, Zelluloseacetat, Polyvinyliden-Fluorid (PVDF) und anderen Fluorpolymeren wie Perfluoralkoxy (PFA) und seinen Derivativen, MFA (Co-Polymer von Tetrafluorethylen und Perfluormethylvinylether), Fluorethylenpropylen-Polymer (FEP) und Kombinationen davon.

6. Nadelfiltervorrichtung nach Anspruch 5, die ferner Folgendes umfasst:
einen zweiten Vorfilter (40), der dem Filtermedium vorgelagert in der Einlassöffnung oder der Filterkammer [071] befestigt ist, wobei der zweite Vorfilter eine oder eine Vielzahl von Schichten aufweist, wobei jede Schicht eine Vielzahl von Poren aufweist, die in der Größe zwischen ungefähr 0,2 µm und ungefähr 5 µm liegen, wobei der zweite Vorfilter aus Fasermaterial gestaltet ist, das Mikrofaser- und Nanofasermaterial und/oder poröse Membran umfasst, ausgewählt aus der Gruppe, die besteht aus Polyethylen, Polypropylen, Nylon, Polyester, Kohlenstoff, Glasfaser, Polypropylensulfid (PPS), Polytetrafluorethylen (Teflon®, PTFE), Polyvinylidenfluorid (PVDF), Polyacrylnitril (PAN), Ethylen-Chlortrifluorethylen (ECTFE), Polyethylen/ultrahochmolekularem Polyethylen (PE/UPE), Zellulose/Kieselgur oder Kieselerdemischungen, Zellulose/Kohlenstoffpartikeln oder -fasern, Zellulose/Ionenaustauschharzen, Zellulose-Acetat, Nitrozellulose, Polyethersulfon, Polysulfon, Zelluloseacetat, Polyvinyliden-Fluorid (PVDF) und anderen Fluorpolymeren wie Perfluoralkoxy (PFA) und seinen Derivativen, MFA (Co-Polymer von Tetrafluorethylen und Perfluormethylvinylether), Fluorethylenpropylen-Polymer (FEP) und Kombinationen davon, und wobei der zweite Vorfilter ausgewählt wird aus der Gruppe, die besteht aus grobporigen Schwämmen, Sieben, Membranen, die eine oder mehrere Schichten, Flachmembranen, tubuläre oder Hohlfasermembranen und Mischungen davon aufweist.

7. Nadelfiltervorrichtung nach Anspruch 1 bis 3, wobei das Filtermedium eine Einzelschicht ist, die mindestens einen hydrophilen Bereich und mindestens einen hydrophoben Bereich aufweist, wobei jeder Bereich sich von einer vorgelagerten Fläche zu einer nachgelagerten Fläche des Filters erstreckt.

8. Nadelfiltervorrichtung nach Anspruch 1 bis 3, wobei das Filtermedium aus der Gruppe ausgewählt ist, die aus Membranen besteht, die eine oder mehrere Schichten, Flachmembranen, tubuläre oder Hohlfasermembranen und Mischungen davon aufweisen, und wobei das Filtermedium eine Vielzahl von Poren aufweist, die in der Größe zwischen ungefähr 0,01 µm und ungefähr 5 µm liegen.

9. Nadelfiltervorrichtung nach Anspruch 1 bis 3, wobei das Filtermedium eine Vielzahl von Membranschichten umfasst, wobei die Schichten alle hydrophil, hydrophob oder Mischungen aus hydrophil und hydrophob sind.

10. Nadelfiltervorrichtung nach Anspruch 9, wobei mindestens eine Schicht der Vielzahl von Membranschichten entweder hydrophil oder hydrophob ist und wobei die mindestens eine Schicht mindestens einen Bereich aufweist, der modifiziert ist, um die gegensätzliche Eigenschaft aufzuweisen.

11. Nadelfiltervorrichtung nach Anspruch 1 bis 3, die ferner eine Halsverlängerung (40) umfasst, die den Hals, der die Spritze aufnimmt, überlagert, wobei die Halsverlängerung im Wesentlichen hohl ist, mit Stufenkonussegmenten (42, 44) von seriell größeren Querschnittsdurchmessern.

12. Nadelfiltervorrichtung nach Anspruch 1 bis 3, die ferner eine Nadel (34) umfasst, die in der Vorrichtung befestigt sind, und eine Nadelansatzkappe (37), die an einem Kappenende (36) der Nadel befestigt ist.

13. Nadelfiltervorrichtung nach Anspruch 1 bis 3, wobei das Filtermedium aus der Gruppe ausgewählt wird, die aus hydrophil, hydrophob und Mischungen daraus besteht, und in der Filterkammer befestigt ist,
wobei der Hals, der die Nadel aufnimmt, ein integraler oder modularer verlängerter Hals ist, der die Nadel aufnimmt, der sich von dem zweiten Ende des Filtergehäuses erstreckt, und wobei der Hals eine obere ringförmige Fläche zum Anlegen an einem Spritzenkörper aufweist.

14. Nadelfiltervorrichtung nach Anspruch 13, wobei eine Außenwand der Einlassöffnung als eine Widerhaken- oder Gleitringfläche ausgelegt ist.

15. Nadelfiltervorrichtung nach Anspruch 14, die ferner eine Spritzennadel umfasst, die an der Einlassöffnung befestigt ist.

## Revendications

1. Appareil (10) à filtre pour aiguille permettant de filtrer des fluides et des solutions lors de leur aspiration dans une seringue (30) avant leur administration à un patient, comprenant :
un logement (14) pour filtre comportant une première extrémité et une seconde extrémité et comportant des parties définissant une chambre de filtration (11) ;
un support de filtre (16) attaché à la chambre de filtration ;
un orifice d'entrée (24) s'étendant à partir du logement pour filtre, l'orifice d'entrée comportant des parties définissant un canal en communication fluidique avec la chambre de filtration ;
un col (12) de réception de seringue s'étendant depuis la seconde extrémité du logement pour filtre, le col comportant des parties définissant une chambre (17) de col en communication fluidique avec la chambre de filtration ;
l'appareil à filtre pour aiguille **se caractérisant par** :
un épaulement (18) d'arrêt d'aiguille attaché à la chambre de col ou formé dans celle-ci, l'épaulement s'étendant radialement vers l'intérieur à partir de la paroi intérieure (13) de col de manière à interagir avec une ligne centrale de la chambre de col et de procurer une barrière mécanique au passage de l'aiguille au-delà de l'épaulement, et l'épaulement ayant une première extrémité qui s'étend vers le bas vers le logement de filtre et à proximité de celui-ci, de manière à créer un espace entre l'épaulement et le logement de filtre, et une seconde extrémité qui s'étend vers le haut le long de la paroi intérieure du col pour former une surface d'ajustement de bouchon ; et
un bouchon (20) de scellement d'aiguille attaché dans le col de réception, le bouchon étant conçu pour s'ajuster contre la surface d'ajustement du bouchon.

2. Appareil à filtre pour aiguille selon la revendication 1, comprenant en outre une bague (22) de retenue de bouchon attachée au col de réception de seringue, la bague comprenant en outre une paroi intérieure présentant une forme tronconique en coupe transversale, et dans lequel la bague s'ajuste contre le bouchon en empêchant le déplacement ou le retrait du bouchon du col.

3. Appareil (10) à filtre pour aiguille permettant de filtrer des fluides et des solutions lors de leur aspiration dans une seringue (30) avant leur administration à un patient, comprenant :
un logement (14) pour filtre comportant une première extrémité et une seconde extrémité et comportant des parties définissant une chambre de filtration (11) ;
un support de filtre (16) attaché à la chambre de filtration ;
l'appareil à filtre pour aiguille **se caractérisant par** :
un orifice d'entrée (24) s'étendant à partir du logement pour filtre, l'orifice d'entrée comportant des parties définissant un canal en communication fluidique avec la chambre de filtration ;
un col (12) de réception de seringue s'étendant depuis la seconde extrémité du logement pour filtre, le col comportant une paroi intérieure (13) définissant une chambre (17) de col en communication fluidique avec la chambre de filtration ;
un épaulement (18) d'arrêt d'aiguille attaché à la chambre de col ou formé dans celle-ci, la butée d'aiguille comprenant une plaque (26) de poussée d'aiguille suspendue sensiblement centralement dans la chambre de col entre une pluralité de colonnes d'espacement (28) et fonctionnant comme une butée d'aiguille pour une aiguille (34) de seringue, les colonnes d'espacement étant attachées à la paroi intérieure ou intégrées à celle-ci, chacune des colonnes d'espacement comprenant une extrémité inférieure (27) s'étendant vers le bas à partir de la plaque de poussée qui forme collectivement une surface d'ajustement destinée à l'ajustement contre les parties du logement de filtre, et une extrémité supérieure (29) s'étendant vers le haut à partir de la plaque de poussée qui forme collectivement une surface d'ajustement destinée à un bouchon (20) de scellement d'aiguille, les extrémités inférieures créant collectivement un espace entre le logement de filtre et un fond de la plaque de poussée, et les extrémités supérieures créant collectivement un espace entre le bouchon de scellement d'aiguille et une surface supérieure de la plaque de poussée, et l'emplacement sensiblement central radial de la plaque de poussée empêchant une aiguille de dépasser la butée d'aiguille quel que soit l'angle ou l'orientation de l'insertion d'aiguille dans l'appareil ; et,
le bouchon de scellement d'aiguille étant attaché dans le col de réception de seringue.

4. Appareil à filtre pour aiguille selon la revendication 3, dans lequel la plaque de poussée comprend une pluralité de régions périmétriques festonnées formées entre les colonnes adjacentes d'espacement de la pluralité de colonnes d'espacement.

5. Appareil à filtre pour aiguille selon la revendication 1 ou 3, comprenant en outre un préfiltre (39) attaché dans l'orifice d'entrée en amont du support de filtre, le préfiltre comportant une pluralité de pores dont la taille est comprise entre environ 10 µm et environ 200 µm, le préfiltre étant choisi dans l'ensemble constitué d'éponges grossières, d'écrans, de membranes comptant au moins une couche, de membranes planes, de membranes à fibres tubulaires ou creuses et de leurs mélanges, et le préfiltre étant constitué de matériau fibreux contenant un matériau de microfibre et de nanofibre choisi dans l'ensemble constitué de polyéthylène, de polypropylène, de nylon, de polyester, de carbone, de fibre de verre, de poly(sulfure de propylène) (PPS), de polytétrafluoroéthylène (Teflon® PTFE), de poly(fluorure de vinylidène) (PVDF), de polyacrylonitrile (PAN), d'éthylène chlorotrifluoroéthylène (ECTFE), de polyéthylène/polyéthylène à masse moléculaire ultra-élevée (PE/UPE), de mélanges de cellulose/terre diatomée ou de silice, de particules ou de fibres de cellulose/carbone, de résines échangeuses de cellulose/d'ions, d'acétate de cellulose, de nitrocellulose, de polyéthersulfone, de polysulfone, d'acétate de cellulose, de poly(fluorure de vinylidène) (PVDF) et d'autres fluoropolymères tels que le perfluoroalcoxy (PFA) et ses dérivés, le MFA (copolymère de tétrafluoroéthylène et perfluorométhyl vinyl éther), polymère d'éthylène propylène fluoré (FEP) et leurs combinaisons.

6. Appareil à filtre pour aiguille selon la revendication 5, comprenant en outre :
un second préfiltre (40) attaché dans l'orifice d'entrée ou dans la chambre de filtration [071] en amont du support pour filtre, le second préfiltre comptant une pluralité de couches avec chaque couche comportant une pluralité de pores dont la taille varie d'environ 0,2 µm à environ 5 µm, le second préfiltre étant constitué de matériau fibreux contenant un matériau à microfibres et à nanofibres et/ou une membrane poreuse choisie dans l'ensemble constitué de polyéthylène, de polypropylène, de nylon, de polyester, de carbone, de fibre de verre, de poly(sulfure de propylène) (PPS), de polytétrafluoroéthylène (Teflon® PTFE), de poly(fluorure de vinylidène) (PVDF), de polyacrylonitrile (PAN), d'éthylène chlorotrifluoroéthylène (ECTFE), de polyéthylène/polyéthylène à masse moléculaire ultra haute (PE/UPE), de mélanges de cellulose/terre diatomée ou de silice, de particules ou de fibres de cellulose/carbone, de résines échangeuses de cellulose/d'ions, d'acétate de cellulose, de nitrocellulose, de polyéthersulfone, de polysulfone, d'acétate de cellulose, de poly(fluorure de vinylidène) (PVDF) et d'autres fluoropolymères tels que le perfluoroalcoxy (PFA) et ses dérivés, le MFA (copolymère de tétrafluoroéthylène et perfluorométhyl vinyl éther), le polymère d'éthylène propylène fluoré (FEP) et leurs combinaisons, et dans lequel le second préfiltre est choisi dans l'ensemble constitué d'éponges grossières, d'écrans, de membranes comptant au moins une couche, de membranes planes, de membranes à fibres tubulaires ou creuses et de leurs mélanges.

7. Appareil à filtre pour aiguille selon la revendication 1 ou 3, dans lequel le support de filtre est une seule couche comptant au moins une partie hydrophile et au moins une partie hydrophobe, chaque section s'étendant d'une surface amont à une surface aval du filtre.

8. Appareil à filtre pour aiguille selon la revendication 1 ou 3, dans lequel le support de filtre est choisi dans l'ensemble constitué de membranes comptant au moins une couche, de membranes planes, de membranes à fibres tubulaires ou creuses et de leurs mélanges, et dans lequel le support de filtre compte une pluralité de pores dont la taille est comprise entre environ 0,01 µm et environ 5 µm.

9. Appareil à filtre pour aiguille selon la revendication 1 ou 3, dans lequel le support de filtre comprend une pluralité de couches de membrane, les couches étant toutes hydrophiles, hydrophobes ou des mélanges de couches hydrophiles et hydrophobes.

10. Appareil à filtre pour aiguille selon la revendication 9, dans lequel au moins une couche de la pluralité de couches de membrane est soit hydrophile soit hydrophobe et où l'au moins une couche compte au moins une section modifiée pour avoir la caractéristique opposée.

11. Appareil à filtre pour aiguille selon la revendication 1 ou 3, comprenant en outre un col d'extension (40) superposé autour du col de réception de la seringue, le col d'extension étant sensiblement creux avec des segments coniques étagés (42, 44) de diamètres de plus en plus grands en coupe transversale.

12. Appareil à filtre pour aiguille selon la revendication 1 ou 3, comprenant en outre une aiguille (34) attachée dans l'appareil et un chapeau d'aiguille-pavillon (37) attaché à une extrémité de pavillon (36) de l'aiguille.

13. Appareil à filtre pour aiguille selon la revendication 1 ou 3, dans lequel le support de filtre est choisi dans l'ensemble constitué de matière hydrophiles, hydrophobes et de leurs combinaisons, et attaché dans la chambre de filtration,
dans lequel le col de réception de seringue est un col de réception intégral ou modulaire étendu de seringue s'étendant à partir de la seconde extrémité du logement de filtre, et dans lequel le col présente une surface annulaire supérieure pour être ajusté contre un cylindre de seringue.

14. Appareil à filtre pour aiguille selon la revendication 13, dans lequel une paroi extérieure de l'orifice d'entrée est conçu sous forme de barbelure ou de surface de joint glissant.

15. Appareil à filtre pour aiguille selon la revendication 14, comprenant en outre une aiguille de seringue attachée à l'orifice d'entrée.
